# EUROPEAN PATENT APPLICATION

(11) **EP 4 521 414 A1**
(43) Date of publication of application: **12.03.2025**
(21) Application number: 24199430.0
(22) Date of filing: 10.09.2024
(51) Int. Cl.: G16H 40/20, G08B 21/24, G16H 40/63

(54) **HYGIENE COMPLIANCE SYSTEM USING AUGMENTED REALITY**

(30) Priority: 11.09.2023 US 202363581904 P
(71) Applicant: OP-Hygiene IP GmbH, 4704 Niederbipp (CH)
(72) Inventor: OPHARDT, Heiner, 4422 Arisdorf (CH); OPHARDT, Joel, Smithville, ON L0R 2A0 (CA); GEURTS, Joshua, Smithville, ON L0R 2A0 (CA)
(74) Representative: Grünecker Patent- und Rechtsanwälte PartG mbB

(57) **Abstract**

A system including an augmented reality device configured to present augmented reality content to a user, and at least one sensor configured to detect a hygiene event. The system is configured to determine a hygiene status of the user based at least in part on data collected by the at least one sensor. The augmented reality content that is presented to the user is based, at least in part, on the hygiene status of the user.

## Description

### Related Application

This application claims priority to the 11 September 2023 filing date of United States Provisional Patent Application Serial No. 63/581,904, which is incorporated herein by reference.

### Field of the Invention

This invention relates to hygiene systems, and more particularly to a system that uses augmented reality to promote hand hygiene.

### Background of the Invention

Poor compliance with hand hygiene protocols in healthcare settings is common, and is a leading cause of hospital or healthcare acquired infections.

Various means of encouraging hand hygiene compliance are known. For example, U.S. Patent No. 10,424,184 to Ophardt, issued September 24, 2019, teaches a monitoring system in which the hand hygiene compliance data of users is collected, and rewards are provided to the users based on their hygiene compliance.

The inventors have appreciated a disadvantage of the prior art is that feedback on hand hygiene compliance is often delayed and may be presented in a manner that users can easily ignore.

### Summary of the Invention

To at least partially overcome some of the disadvantages of previously known systems, devices and methods, in one aspect the present invention provides a system including an augmented reality device configured to present augmented reality content to a user, and at least one sensor configured to detect a hygiene event. The system is configured to determine a hygiene status of the user based at least in part on data collected by the at least one sensor. The augmented reality content that is presented to the user is based, at least in part, on the hygiene status of the user.

The inventors have appreciated that providing hygiene information to a user through augmented reality allows the information to be provided in real time, thereby allowing the user to modify their behavior in the moment based on the real time feedback. Furthermore, augmented reality allows the information to be provided in a visually striking manner that is difficult to ignore.

For example, the augmented reality device may be in the form of a headset or smart glasses that displays visual content overlaid onto the visual field of the user. This preferably makes the information difficult to ignore, as compared for example to an alert on the user's phone, which may be in the user's pocket or otherwise out of sight.

Preferably, the system is configured to provide clear and unambiguous feedback about the hygiene status of the user, and the actions that are permitted and/or prohibited based on that hygiene status. For example, in a hospital setting, a healthcare worker may be required to sanitize her hands each time she enters a new patient's room, and before she approaches the patient's bed. Preferably, the system is configured to detect whether or not the healthcare worker has properly sanitized her hands, and to provide real time feedback to guide the worker's behavior.

For example, if the system determines that the worker has not yet sanitized her hands, the augmented reality device may provide a visual alert in the form of a visual highlight that surrounds the patient's bed in the worker's field of view. The highlight may, for example, be in the form of a red light or tint, indicating that the worker is not permitted to approach the patient's bed.

The red light will preferably remind the worker that she needs to sanitize her hands before approaching the patient's bed. Optionally, additional cues may be provided to guide the worker's behavior, such as text or video instructions which may be displayed in the user's visual field. The augmented reality device can also be configured to highlight the location of the nearest sanitation station, for example by highlighting the station in blue.

The system is preferably configured to determine whether the worker has complied with a hand hygiene protocol based on data from the at least one sensor. The at least one sensor may, for example, be a sensor in the sanitation station that detects a sanitation event. The sensor may, for example, be configured to detect when hand sanitizer or hand soap has been dispensed onto the worker's hands and/or the volume of hand sanitizer or hand soap that has been dispensed. This information is then used by the system to determine whether or not the hygiene protocol has been complied with.

Once the system determines that the worker has complied with the hand hygiene protocol, the augmented reality content is modified to indicate to the worker that she can now approach the patient's bed by removing the red highlight. Optionally, other cues could be provided to inform the worker that the protocol has been complied with, such as temporarily highlighting the patient's bed in green, or temporally displaying a green check mark in the worker's visual field.

Further aspects of the invention include:
A system, which optionally incorporates one or more features of any one or more of the following and/or preceding aspects, comprising: an augmented reality device configured to present augmented reality content to a user; and at least one sensor configured to detect a hygiene event; wherein the system is configured to determine a hygiene status of the user based at least in part on data collected by the at least one sensor; and wherein at least some of the augmented reality content is selected based on the hygiene status of the user.

A system, which optionally incorporates one or more features of any one or more of the following and/or preceding aspects, wherein the augmented reality content comprises visual content that is overlaid onto a visual field of the user.

A system, which optionally incorporates one or more features of any one or more of the following and/or preceding aspects, wherein the visual content comprises a visual highlight of a feature in the visual field of the user.

A system, which optionally incorporates one or more features of any one or more of the following and/or preceding aspects, wherein the visual highlight comprises a color that is overlaid onto the feature.

A system, which optionally incorporates one or more features of any one or more of the following and/or preceding aspects, wherein the system is configured to determine whether the user is permitted to perform an action related to the feature based at least in part on the hygiene status of the user; and wherein, when the user is prohibited from performing the action, the visual highlight is presented to alert the user that the user is prohibited from performing the action.

A system, which optionally incorporates one or more features of any one or more of the following and/or preceding aspects, wherein the feature comprises a physical space and the interaction comprises entering the physical space.

A system, which optionally incorporates one or more features of any one or more of the following and/or preceding aspects, wherein the physical space comprises a patient zone and the user is a health care worker.

A system, which optionally incorporates one or more features of any one or more of the following and/or preceding aspects, wherein the feature comprises an object and the interaction comprises touching the object.

A system, which optionally incorporates one or more features of any one or more of the following and/or preceding aspects, wherein the feature comprises a person and the interaction comprises touching the person.

A system, which optionally incorporates one or more features of any one or more of the following and/or preceding aspects, wherein the person is a patient and the user is a health care worker.

A system, which optionally incorporates one or more features of any one or more of the following and/or preceding aspects, wherein the system is configured to: determine, based at least in part on the data collected by the at least one sensor, when the hygiene status of the user has changed such that the user is now permitted to perform the action related to the feature; and modify the visual content to communicate to the user that the user is now permitted to perform the action.

A system, which optionally incorporates one or more features of any one or more of the following and/or preceding aspects, comprising: an augmented reality device configured to present augmented reality content to a user; and at least one sensor configured to detect a characteristic of a dispenser; wherein at least some of the augmented reality content is selected based on the characteristic of the dispenser.

A system, which optionally incorporates one or more features of any one or more of the following and/or preceding aspects, wherein the augmented reality content comprises visual content that is overlaid onto a visual field of the user.

A system, which optionally incorporates one or more features of any one or more of the following and/or preceding aspects, wherein the characteristic comprises a fill level of the dispenser.

A system, which optionally incorporates one or more features of any one or more of the following and/or preceding aspects, wherein the system is configured to determine whether the dispenser needs to be refilled; and when the dispenser needs to be refilled, the visual content comprises a visual highlight of the dispenser.

A system, which optionally incorporates one or more features of any one or more of the following and/or preceding aspects, comprising: an augmented reality device configured to present augmented reality content to a user; and at least one sensor configured to detect a hygiene event; wherein the system is configured to determine a hygiene status of the user based at least in part on data collected by the at least one sensor; and wherein at least some of the augmented reality content is selected based on the hygiene status of the user.

A system, which optionally incorporates one or more features of any one or more of the following and/or preceding aspects, wherein the augmented reality content comprises visual content that is overlaid onto a visual field of the user.

A system, which optionally incorporates one or more features of any one or more of the following and/or preceding aspects, wherein the visual content comprises a visual highlight of a feature in the visual field of the user.

A system, which optionally incorporates one or more features of any one or more of the following and/or preceding aspects, wherein the visual highlight comprises a color that is overlaid onto the feature.

A system, which optionally incorporates one or more features of any one or more of the following and/or preceding aspects, wherein the system is configured to determine whether the user is permitted to perform an action related to the feature based at least in part on the hygiene status of the user.

A system, which optionally incorporates one or more features of any one or more of the following and/or preceding aspects, wherein, when the user is permitted to perform the action, the visual highlight is selected to inform the user that the user is permitted to perform the action.

A system, which optionally incorporates one or more features of any one or more of the following and/or preceding aspects, wherein, when the user is prohibited from performing the action, the visual highlight is selected to inform the user that the user is prohibited from performing the action.

A system, which optionally incorporates one or more features of any one or more of the following and/or preceding aspects, wherein the feature comprises a physical space and the action comprises entering the physical space.

A system, which optionally incorporates one or more features of any one or more of the following and/or preceding aspects, wherein the physical space comprises a patient zone and the user is a health care worker.

A system, which optionally incorporates one or more features of any one or more of the following and/or preceding aspects, wherein the feature comprises an object and the action comprises touching the object.

A system, which optionally incorporates one or more features of any one or more of the following and/or preceding aspects, wherein the feature comprises a person and the action comprises touching the person.

A system, which optionally incorporates one or more features of any one or more of the following and/or preceding aspects, wherein the person is a patient and the user is a health care worker.

A system, which optionally incorporates one or more features of any one or more of the following and/or preceding aspects, wherein the system is configured to: determine, based at least in part on the data collected by the at least one sensor, when the hygiene status of the user has changed such that the user is now permitted to perform the action related to the feature; and modify the visual content to communicate to the user that the user is now permitted to perform the action.

A system, which optionally incorporates one or more features of any one or more of the following and/or preceding aspects, wherein, when the user is prohibited from performing the action, the augmented reality device is configured to produce an alert if the user approaches the feature.

A system, which optionally incorporates one or more features of any one or more of the following and/or preceding aspects, wherein the alert comprises a modification of the visual highlight.

A system, which optionally incorporates one or more features of any one or more of the following and/or preceding aspects, wherein the alert comprises an audible warning.

A system, which optionally incorporates one or more features of any one or more of the following and/or preceding aspects, wherein the augmented reality device comprises a headset that is worn by the user.

A system, which optionally incorporates one or more features of any one or more of the following and/or preceding aspects, wherein the at least one sensor is incorporated into the augmented reality device.

A system, which optionally incorporates one or more features of any one or more of the following and/or preceding aspects, wherein the at least one sensor is incorporated into a hand cleaning fluid dispenser.

A system, which optionally incorporates one or more features of any one or more of the following and/or preceding aspects, wherein the system is configured to determine whether the user should clean their hands; and wherein, when the system determines that the user should clean their hands, the augmented reality content comprises content to encourage the user to clean their hands.

A system, which optionally incorporates one or more features of any one or more of the following and/or preceding aspects, wherein the content to encourage the user to clean their hands comprises a visual highlight of a hand cleaning station in the visual field of the user.

A system, which optionally incorporates one or more features of any one or more of the following and/or preceding aspects, wherein the system is configured to determine, based at least in part on the data collected by the at least one sensor, whether the user has complied with a hand hygiene protocol.

A system, which optionally incorporates one or more features of any one or more of the following and/or preceding aspects, wherein the system is configured to track a location of the user; and wherein the augmented reality content is modified based on the location of the user.

A system, which optionally incorporates one or more features of any one or more of the following and/or preceding aspects, wherein the system is configured to determine whether the user should put on personal protective equipment; and wherein, when the system determines that the user should put on personal protective equipment, the augmented reality content comprises content to encourage the user to put on personal protective equipment.

A system, which optionally incorporates one or more features of any one or more of the following and/or preceding aspects, wherein the content to encourage the user to put on personal protective equipment comprises a visual highlight of a personal protective equipment station in the visual field of the user.

A system, which optionally incorporates one or more features of any one or more of the following and/or preceding aspects, wherein the hygiene event comprises hand cleaning.

A system, which optionally incorporates one or more features of any one or more of the following and/or preceding aspects, wherein the hygiene event comprises dispensing a hand cleaning fluid.

A system, which optionally incorporates one or more features of any one or more of the following and/or preceding aspects, wherein the hygiene event comprises entering a patient room.

A system, which optionally incorporates one or more features of any one or more of the following and/or preceding aspects, wherein the hygiene event comprises entering a bathroom.

A system, which optionally incorporates one or more features of any one or more of the following and/or preceding aspects, wherein the hygiene event comprises touching a patient.

A system, which optionally incorporates one or more features of any one or more of the following and/or preceding aspects, wherein the hygiene event comprises putting on personal protective equipment.

A system, which optionally incorporates one or more features of any one or more of the following and/or preceding aspects, wherein the hygiene event comprises a failure to comply with a hand hygiene protocol.

A system, which optionally incorporates one or more features of any one or more of the following and/or preceding aspects, wherein the hygiene event comprises a failure to perform hand cleaning.

A system, which optionally incorporates one or more features of any one or more of the following and/or preceding aspects, wherein the feature comprises the user's hands.

A system, which optionally incorporates one or more features of any one or more of the following and/or preceding aspects, wherein the action comprises using the user's hands to touch a person and/or an object.

A method, which optionally incorporates one or more features of any one or more of the following and/or preceding aspects, comprising: determining a hygiene status of an individual; and displaying augmented reality content to the individual based on the hygiene status of the individual.

A method, which optionally incorporates one or more features of any one or more of the following and/or preceding aspects, further comprising: detecting a hygiene event; wherein the hygiene status of the individual is determined, at least in part, based on the hygiene event.

A method, which optionally incorporates one or more features of any one or more of the following and/or preceding aspects, wherein the method is performed using the system in accordance with any one or more of the preceding and/or following aspects.

A system, which optionally incorporates one or more features of any one or more of the following and/or preceding aspects, comprising: an augmented reality device configured to present augmented reality content to a user; and at least one sensor configured to detect a characteristic of a dispenser; wherein at least some of the augmented reality content is selected based on the characteristic of the dispenser.

A system, which optionally incorporates one or more features of any one or more of the following and/or preceding aspects, wherein the augmented reality content comprises visual content that is overlaid onto a visual field of the user.

A system, which optionally incorporates one or more features of any one or more of the following and/or preceding aspects, wherein the characteristic comprises a fill level of the dispenser.

A system, which optionally incorporates one or more features of any one or more of the following and/or preceding aspects, wherein the system is configured to determine whether the dispenser needs to be refilled; and when the dispenser needs to be refilled, the visual content comprises a visual highlight of the dispenser.

A system, which optionally incorporates one or more features of any one or more of the following and/or preceding aspects, wherein the dispenser comprises a hygiene product dispenser.

A system, which optionally incorporates one or more features of any one or more of the following and/or preceding aspects, wherein the dispenser comprises a hand cleaning fluid dispenser.

A system, which optionally incorporates one or more features of any one or more of the following and/or preceding aspects, wherein the at least one sensor comprises a fill level sensor that is incorporated into the dispenser.

A system, which optionally incorporates one or more features of any one or more of the following and/or preceding aspects, comprising: an augmented reality device configured to present augmented reality content to a user; and at least one sensor configured to detect a sanitation event; wherein the system is configured to determine a sanitation status of an object based at least in part on data collected by the at least one sensor; and wherein at least some of the augmented reality content is selected based on the sanitation status of the object.

A system, which optionally incorporates one or more features of any one or more of the following and/or preceding aspects, wherein the augmented reality content comprises visual content that is overlaid onto a visual field of the user.

A system, which optionally incorporates one or more features of any one or more of the following and/or preceding aspects, wherein the visual content comprises a visual highlight of the object or a portion of the object in the visual field of the user.

A system, which optionally incorporates one or more features of any one or more of the following and/or preceding aspects, wherein the visual highlight comprises a color that is overlaid onto the object or the portion of the object.

A system, which optionally incorporates one or more features of any one or more of the following and/or preceding aspects, wherein the sanitation event comprises cleaning the object.

A system, which optionally incorporates one or more features of any one or more of the following and/or preceding aspects, wherein the augmented reality content comprises a visual highlight of a portion of the object that has not yet been cleaned.

A system, which optionally incorporates one or more features of any one or more of the following and/or preceding aspects, wherein the object comprises a floor.

A system, which optionally incorporates one or more features of any one or more of the following and/or preceding aspects, wherein the object comprises a wall.

A system, which optionally incorporates one or more features of any one or more of the following and/or preceding aspects, wherein the object comprises a door.

A system, which optionally incorporates one or more features of any one or more of the following and/or preceding aspects, wherein the object comprises a bed.

A system, which optionally incorporates one or more features of any one or more of the following and/or preceding aspects, wherein the object comprises a room.

A system, which optionally incorporates one or more features of any one or more of the following and/or preceding aspects, wherein the object comprises a toilet.

A system, which optionally incorporates one or more features of any one or more of the following and/or preceding aspects, wherein the object comprises a patient's body.

A system, which optionally incorporates one or more features of any one or more of the following and/or preceding aspects, wherein the object comprises the user's body.

A system, which optionally incorporates one or more features of any one or more of the following and/or preceding aspects, wherein the at least one sensor comprises a camera that is incorporated into the augmented reality device.

A system, which optionally incorporates one or more features of any one or more of the following and/or preceding aspects, comprising: an augmented reality device configured to present augmented reality content to a user; and at least one sensor configured to detect a contamination event; wherein the system is configured to determine a contamination status of an object based at least in part on data collected by the at least one sensor; and wherein at least some of the augmented reality content is selected based on the contamination status of the object.

A system, which optionally incorporates one or more features of any one or more of the following and/or preceding aspects, wherein the augmented reality content comprises visual content that is overlaid onto a visual field of the user.

A system, which optionally incorporates one or more features of any one or more of the following and/or preceding aspects, wherein the visual content comprises a visual highlight of the object or a portion of the object in the visual field of the user.

A system, which optionally incorporates one or more features of any one or more of the following and/or preceding aspects, wherein the visual highlight comprises a color that is overlaid onto the object or the portion of the object.

A system, which optionally incorporates one or more features of any one or more of the following and/or preceding aspects, wherein the contamination event comprises a person touching the object.

A system, which optionally incorporates one or more features of any one or more of the following and/or preceding aspects, wherein the system is configured to determine a hygiene status of the person.

A system, which optionally incorporates one or more features of any one or more of the following and/or preceding aspects, wherein the augmented reality content is selected based, at least in part, on the hygiene status of the person.

A system, which optionally incorporates one or more features of any one or more of the following and/or preceding aspects, wherein the hygiene status of the person is determined based, at least in part, on whether the person has complied with a hand hygiene protocol.

A system, which optionally incorporates one or more features of any one or more of the following and/or preceding aspects, wherein the at least one sensor comprises a camera.

A system, which optionally incorporates one or more features of any one or more of the following and/or preceding aspects, wherein the at least one sensor comprises a camera that is incorporated into the augmented reality device.

A system, which optionally incorporates one or more features of any one or more of the following and/or preceding aspects, further comprising a plurality of additional augmented reality devices that are configured to present augmented reality content to additional users.

A system, which optionally incorporates one or more features of any one or more of the following and/or preceding aspects, wherein the at least one sensor comprises at least one camera that is incorporated into one or more of the additional augmented reality devices.

A system, which optionally incorporates one or more features of any one or more of the following and/or preceding aspects, wherein the system is configured to track a plurality of said contamination events over time; and wherein the augmented reality content is selected based, at least in part, on the number of said contamination events detected over time.

A system, which optionally incorporates one or more features of any one or more of the following and/or preceding aspects, wherein the system is configured to determine whether the object should be cleaned.

A system, which optionally incorporates one or more features of any one or more of the following and/or preceding aspects, wherein the system is configured to notify a cleaner when it is determined that the object should be cleaned.

A system, which optionally incorporates one or more features of any one or more of the following and/or preceding aspects, wherein the system is configured to detect a cleaning event.

A system, which optionally incorporates one or more features of any one or more of the following and/or preceding aspects, wherein the augmented reality content is modified when a cleaning event is detected.

A system, which optionally incorporates one or more features of any one or more of the following and/or preceding aspects, wherein the contamination event comprises a possible exposure of the object to a pathogen.

A system, which optionally incorporates one or more features of any one or more of the following and/or preceding aspects, comprising: an augmented reality device configured to present augmented reality content to a user; and at least one sensor configured to detect a hygiene activity of a person; wherein the system is configured to determine a hygiene status of the person based at least in part on data collected by the at least one sensor; and wherein at least some of the augmented reality content is selected based on the hygiene status of the person.

A system, which optionally incorporates one or more features of any one or more of the following and/or preceding aspects, wherein the augmented reality content comprises visual content that is overlaid onto a visual field of the user.

A system, which optionally incorporates one or more features of any one or more of the following and/or preceding aspects, wherein the visual content comprises a visual highlight of the person in the visual field of the user.

A system, which optionally incorporates one or more features of any one or more of the following and/or preceding aspects, wherein the visual highlight comprises a color that is overlaid onto the person.

A system, which optionally incorporates one or more features of any one or more of the following and/or preceding aspects, wherein the hygiene event comprises a failure to comply with a hand hygiene protocol.

A system, which optionally incorporates one or more features of any one or more of the following and/or preceding aspects, wherein the augmented reality content is selected to inform the user that the person is in breach of the hand hygiene protocol.

A system, which optionally incorporates one or more features of any one or more of the following and/or preceding aspects, wherein the visual content comprises a visual highlight of the user's hands in the visual field of the user.

A system, which optionally incorporates one or more features of any one or more of the following and/or preceding aspects, comprising: an augmented reality device configured to present augmented reality content to a user; and at least one sensor configured to detect an infection status of a person; wherein at least some of the augmented reality content is selected based on the infection status of the person.

A system, which optionally incorporates one or more features of any one or more of the following and/or preceding aspects, wherein the augmented reality content comprises visual content that is overlaid onto a visual field of the user.

A system, which optionally incorporates one or more features of any one or more of the following and/or preceding aspects, wherein the visual content comprises a visual highlight of the person in the visual field of the user.

A system, which optionally incorporates one or more features of any one or more of the following and/or preceding aspects, wherein the visual highlight comprises a color that is overlaid onto the person.

A system, which optionally incorporates one or more features of any one or more of the following and/or preceding aspects, wherein the augmented reality content is selected to inform the user that the person has an infection or a possible infection.

A system, which optionally incorporates one or more features of any one or more of the following and/or preceding aspects, wherein the at least one sensor is incorporated into a hand cleaning fluid dispenser.

A system, which optionally incorporates one or more features of any one or more of the following and/or preceding aspects, wherein the at least one sensor comprises a touchless thermometer.

A system, which optionally incorporates one or more features of any one or more of the following and/or preceding aspects, wherein the at least one sensor comprises a biological particle sensor.

A method, which optionally incorporates one or more features of any one or more of the following and/or preceding aspects, comprising: determining a characteristic of a dispenser; and displaying augmented reality content to an individual based on the characteristic of the dispenser.

A method, which optionally incorporates one or more features of any one or more of the following and/or preceding aspects, comprising: determining a sanitation status of an object; and displaying augmented reality content to an individual based on the sanitation status of the object.

A method, which optionally incorporates one or more features of any one or more of the following and/or preceding aspects, comprising: determining a contamination status of an object; and displaying augmented reality content to an individual based on the contamination status of the object.

A method, which optionally incorporates one or more features of any one or more of the following and/or preceding aspects, comprising: determining a hygiene status of a person; and displaying augmented reality content to an individual based on the hygiene status of the person.

A method, which optionally incorporates one or more features of any one or more of the following and/or preceding aspects, comprising: determining an infection status of a person; and displaying augmented reality content to an individual based on the infection status of the person.

### Brief Description of the Drawings

Further aspects and advantages of the invention will appear from the following description taken together with the accompanying drawings, in which:
Figure 1 is a perspective view of a healthcare worker using a hygiene compliance system in accordance with a first embodiment of the present invention;
Figure 2 is a representation of a field of view of the healthcare worker shown in Figure 1, as viewed through an augmented reality headset, while the healthcare worker is looking at a patient's bed; and
Figure 3 is a representation of a field of view of the healthcare worker shown in Figure 1, as viewed through the augmented reality headset, while the healthcare worker is looking at a hand sanitizer dispenser.

### Detailed Description of the Drawings

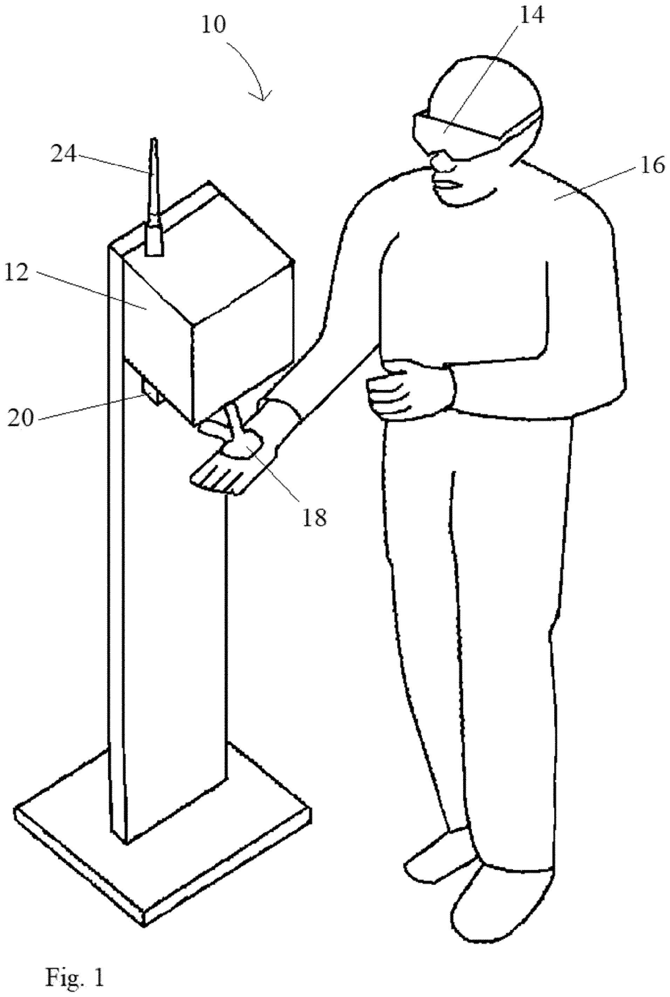

Figure 1 shows a hygiene compliance system 10 in accordance with a first embodiment of the present invention. The system 10 includes at least one hand cleaning fluid dispenser 12 and at least one augmented reality device 14 that is worn by at least one user 16. For simplicity, Figure 1 shows only a single hand cleaning fluid dispenser 12 and a single augmented reality device 14, but preferably the system 10 would include a plurality of dispensers 12 positioned at different locations in a facility, and a plurality of augmented reality devices 14 worn by a plurality of users 16.

The fluid dispenser 12 is configured to dispense hand cleaning fluid 18 onto the hand of the user 16, which may be used to clean and/or sanitize the user's hands in accordance with a hygiene protocol that has been established for the user and/or for the facility where the system 10 is being used. For example, the system 10 might be used in a hospital or other healthcare facility where the hand hygiene of staff and/or visitors is being monitored in order to reduce the spread of infections within the facility.

The fluid dispenser 12 may have any suitable structure for dispensing the hand cleaning fluid 18, such as those disclosed in United States Patent No. 8,245,877 to Ophardt, issued August 21, 2012; United States Patent No. 8,113,388 to Ophardt et al., issued February 14, 2012; United States Patent No. 8,091,739 to Ophardt et al., issued January 10, 2012; United States Patent No. 7,748,573 to Anhuf et al., issued July 6, 2010; U.S. Patent No. 7,984,825 to Ophardt et al., issued July 26, 2011; U.S. Patent No. 8,684,236 to Ophardt, issued April 1, 2014; U.S. Patent No. 5,373,970 to Ophardt, issued December 20, 1994; U.S. Patent No. 5,836,482 to Ophardt et al., issued November 17, 1998; and U.S. Patent No. 9,682,390 to Ophardt et al., issued June 20, 2017, which are incorporated herein by reference.

Preferably, the dispenser 12 includes a sensor 20 that is configured to detect when hand cleaning fluid 18 has been dispensed onto the user's hand, in order to determine whether the user 16 is in compliance with a hand hygiene protocol. For example, the sensor 20 may be in the form of a contactless infrared thermometer, which detects a change in temperature in order to determine when fluid 18 has been dispensed onto the user's hand, as is disclosed in U.S. Patent No. 11,622,656 to Ophardt, issued April 11, 2023, which is incorporated herein by reference.

The dispenser 12 also preferably has a communication device 24 for communicating information about the hand hygiene of the user 16 to other components of the hygiene compliance system 10. For example, the dispenser 12 may be configured to communicate directly with the augmented reality device 14 and/or with a central server.

The augmented reality device 14 is in the form of a headset that is worn by the user 16. The headset at least partially covers the user's eyes and has one or more displays that present augmented reality content to the user 16. The augmented reality device 14 preferably allows the user 16 to see their physical surroundings, while also displaying computer generated visual content that is overlaid onto the user's visual field. The augmented reality device 14 may optionally incorporate one or more transparent displays or heads-up displays. The augmented reality device 14 may also incorporate one or more cameras that record video images of the user's surroundings. These videos can then be modified to incorporate augmented reality content, and presented to the user 16 via the displays. Examples of augmented reality devices 14 that might be used with the invention include Google Glass^{™}, Apple Vision Pro^{™}, and the devices described in U.S. Patent No. 10,078,917 to Gaeta et al., issued September 18, 2018; U.S. Patent No. 10,816,807 to Polcak et al., issued October 27, 2020; U.S. Patent No. 11,363,349 to DeFaria et al., issued June 14, 2022; and U.S. Patent No. 11,372,230 to Peyman, issued June 28, 2022, which are incorporated herein by reference.

The system 10 is preferably configured to display augmented reality content based on the hygiene status of the user 16. For example, the system 10 may be configured to display visual content alerting the user 16 of activities that are permitted or prohibited based on their hygiene status.

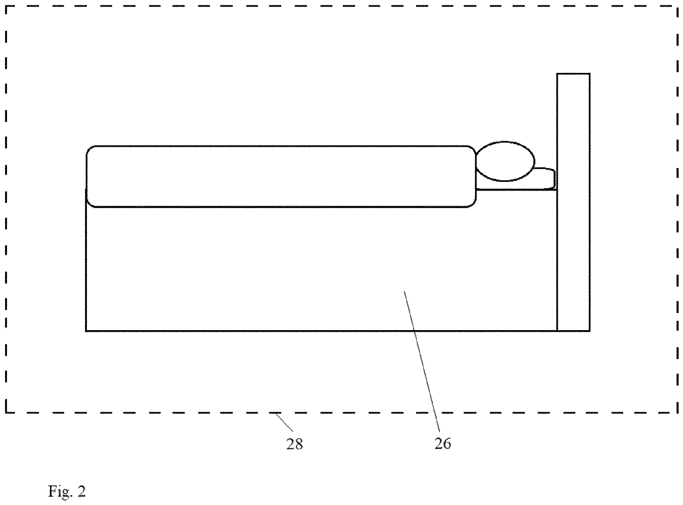

As an example, the system 10 may be configured to monitor and encourage compliance with a hygiene protocol in which healthcare workers are required to sanitize their hands after they enter a patient's room, and before they approach or touch the patient. An example of the visual content that could be displayed in this case is shown in Figure 2.

The system 10 is preferably configured to determine whether or not the worker has sanitized their hands in accordance with the hygiene protocol based, for example, on data collected by a sensor 20 on a fluid dispenser 12 located in the patient's room. If the sensor 20 has not detected the dispensing of hand cleaning fluid 18 onto the worker's hand, then the system 10 may be configured to determine that the worker is not permitted to approach the patient's bed 26 under the hygiene protocol.

The augmented reality device 14 is preferably configured to communicate this information to the worker by presenting augmented reality content. For example, the augmented reality device 14 may display a visual highlight 28 that is overlaid on top of the patient's bed 26 in the worker's visual field. The visual highlight 28 may, for example, be in the form of a color or tint, such as red, that communicates to the worker that they are not permitted to approach the patient's bed 26.

Optionally, the augmented reality device 14 may provide additional instructions informing the worker that they need to sanitize their hands, for example by displaying text instructions in the worker's visual field and/or by providing audible instructions via a built in speaker.

Optionally, if the worker approaches the patient's bed 26, the augmented reality content could become more prominent. For example, the red tint could begin flashing and/or a loud warning sound could be produced. If the worker comes within a prohibited distance of the patient's bed 26 or touches the patient, the system 10 preferably records this breach of the hygiene protocol. Appropriate action can then be taken, such as providing further training to the worker or taking other corrective action.

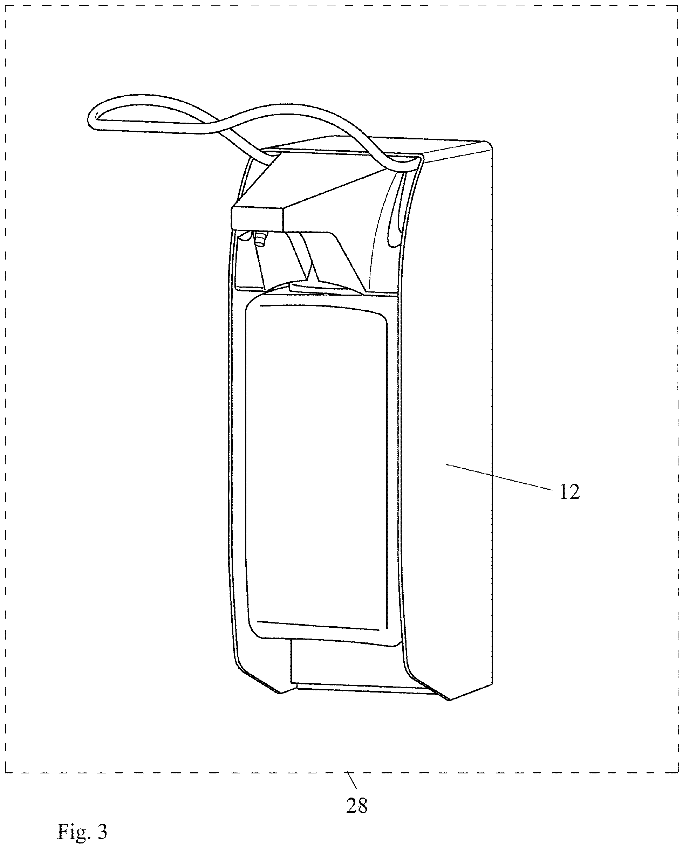

Preferably, the warnings provided by the augmented reality device 14 are sufficient to remind the worker of the need to sanitizer their hands, and they do so by approaching and activating the dispenser 12. Optionally, the system 10 may be configured to assist the worker in locating the dispenser 12 by visually highlighting the dispenser 12, as shown in Figure 3. The visual highlight 28 may, for example, be in the form of a green or blue tint.

When fluid 18 is dispensed onto the worker's hand, this is detected by the sensor 20. Based in the detection data from the sensor 20, the system 10 is able to determine that the worker has now complied with the hygiene protocol, and is permitted to approach the patient's bed 26. The system 10 preferably modifies the augmented reality content in view of this change in the worker's hygiene status. For example, the system 10 will remove the red visual highlight 28 from the patient's bed 26. Other augmented reality content, such as a temporary green tint around the patient's bed, a temporarily visible check mark, and/or an audible confirmation of compliance with the hygiene protocol, could also be provided.

Preferably, the system 10 makes use of real time location information in order to provide accurate compliance monitoring and feedback. For example, the augmented reality device 14 may include a location tracker that determines the user's location as the user 16 travels through the facility. The location tracker may be used, for example, to determine when the user 16 has entered or exited a patient's room, or the location of the user 16 when a hygiene event takes place. The augmented reality device 14 may also be configured to communicate with nearby dispensers 12, in order to detect when the user 16 is in proximity to a dispenser 12. Optionally, the location tracker could also be a separate device that is carried by the user 16, such as a smart phone with built in location tracking capabilities.

The patient and/or the patient's bed 26 could also be provided with devices and/or tags that assist the system 10 in determining the location of the patient and/or the patient's bed 26. For example, the augmented reality device 14 may be configured to detect an RFID tag attached to the patient's bed 26 in order to determine the location of the bed 26 within the user's visual field, so that the appropriate area of the visual field can be highlighted. Optionally, the system 10 may be used in concert with a Real-Time Locating System (RTLS), which tracks the location of various objects and people within the facility.

The system 10 may also be configured to rely on information collected by the augmented reality device 14 in order to determine the location of the user 16, the dispensers 12, the patient's bed 26, and/or other objects or people. For example, the system 10 could employ artificial intelligence that is able to interpret video data recorded by the augmented reality device 14 in order to determine the location of the user 16 in real time, for example by comparing the video data to a digital three dimensional model of the facility. The system 10 could also use artificial intelligence to identify different objects in the video data, such as fluid dispensers 12, beds 26, and/or patient wristbands.

Optionally, the system 10 is configured to receive information from a variety of different sources. For example, hospital administrators may be able to input data regarding different infections that are present in the hospital, and the system 10 may be able to alter the augmented reality content based on this information. As an example, the augmented reality device 14 may be configured to provide enhanced warnings when the user 16 is in the vicinity of a patient who has an antibiotic resistant infection. The enhanced warnings might include, for example, visual content overlaid on the door to a patient's room, warning the user 16 not to enter the room without first donning appropriate personal protective equipment.

In some embodiments of the invention, one or more of the dispensers 12 in the facility may be equipped with sensors that detect and/or identity infections, such as those disclosed in U.S. Patent Publication No. 2022/0091011 to Steltenkamp et al., published March 24, 2022, which is incorporated herein by reference. Optionally, the augmented reality device 14 may be configured to display augmented reality content based on infection data received from the dispensers 12. For example, if the dispenser 12 detects that the user 16 has a possible infection, this information may be displayed as a text warning in the user's visual field. As another example, if the dispenser 12 detects the presence of a pathogen of concern, such as an antibiotic resistant bacteria, the augmented reality device 14 may display this information, and direct the user 16 to take additional steps for enhanced disinfection. The augmented reality device 14 could, for example, highlight a path for the user 16 to follow to reach a dispenser 12 containing a more preferred cleaning fluid 18 for killing the detected pathogen.

In another embodiment of the invention, the system 10 could be adapted for use by maintenance staff in a facility having a plurality of dispensers 12. For example, the system 10 could be configured to receive information from the dispensers 12 regarding the volume of fluid 18 remaining in the dispensers 12, and the augmented reality device 14 could be configured to display augmented reality content that communicates this information to maintenance staff. As an example, when maintenance staff view a dispenser 12 through the augmented reality device 14, a fill level indicator could be displayed adjacent to the dispenser 12 in the user's field of view. This would preferably assist the maintenance personnel in quickly determining which dispensers 12 need to be refilled, without requiring the personnel to closely examine the dispensers 12.

When a dispenser 12 needs to be refilled, a prominent visual display could be provided, such as a blue visual highlight 28 over the dispenser 12. When a dispenser 12 is completely empty, a further enhanced visual display could be provided, such a red and/or flashing visual highlight 28.

In some embodiments of the invention, the system 10 could be configured to determine the optimal routes for maintenance personnel to travel through the facility, such as is disclosed in U.S. Patent No. 10,891,596 to Ophardt et al., issued January 12, 2021, which is incorporated herein by reference. Optionally, the augmented reality device 14 could be configured to assist staff in following the optimal route by highlighting the route in the user's visual field.

It will be understood that, although various features of the invention have been described with respect to one or another of the embodiments of the invention, the various features and embodiments of the invention may be combined or used in conjunction with other features and embodiments of the invention as described and illustrated herein.

Although the system 10 has been described as presenting visual highlights 28, in the form of colors or tints, other types of augmented reality content could also be presented. For example, when a patient is not approachable, a symbol such as an exclamation mark or an image of a hand being cleaned could be displayed on top of or beside the patient. Additional information, such as information from the patient's chart, could also be displayed via augmented reality. Optionally, the chart information could be made inaccessible until hand hygiene protocols have been followed.

The system 10 could be used to provide information, instructions, and/or feedback through augmented reality regarding a wide variety of different individuals and/or objects. The augmented reality system 10 could, for example, provide feedback to a healthcare worker regarding rooms that the worker is permitted or prohibited from entering, or equipment that the worker is permitted or prohibited from touching, based on the worker's hygiene status and/or based on other criteria.

In some embodiments of the invention, the system 10 may employ facial recognition in order to identify different individuals, including distinguishing patients from non-patients. The system 10 could also detect identifying tags and/or badges, such as ID tags worn by healthcare workers and/or wristbands with embedded tags worn by patients. The system 10 may also receive and display information from patient occupancy sensors.

The system 10 may receive data from a variety of different sources, and use this data for a variety of purposes such as monitoring hygiene compliance, selecting and presenting augmented reality content, and modifying augmented reality content. For example, the system 10 may receive data from smart dispensers 12, a Real-Time Locating System (RTLS), people counters, and/or various sensors located around the facility, as well as sensors on the augmented reality device 14 itself.

Although the preferred embodiments have been described as using data from a sensor 20 located on a fluid dispenser 12 in order to determine the hygiene status of the user 16, this is not required. Rather, any suitable sensor capable of determining the hygiene status of the user 16 could be used. For example, the augmented reality device 14 could be configured to record video using a camera or optical sensor, and the system 10 could analyze this video in order to determine the hygiene status of the user 16. The system 10 may, for example, be configured to recognize when the user 16 has dispensed hand cleaning fluid 18 onto the user's hands based on the recorded video. The system 10 could also be configured to analyze the hygiene event to assess whether the proper hygiene protocol has been followed, such as the volume of fluid 18 dispensed, the duration of the hand scrubbing motions, and/or the adequacy of the scrubbing motions. Optionally, the augmented reality device 14 could be used to display instructional videos showing the proper hand scrubbing technique. This could be triggered, for example, when the system 10 detects that the scrubbing motions being used by the user 16 are inadequate.

The system 10 is not limited to assessing and providing feedback on hand cleaning, and may be configured to assess and provide feedback on other hygiene related actions such as wearing gloves, masks, or other personal protective equipment.

The system 10 may be configured to visually identify, via augmented reality: a patient from a non-patient; the distance to the patient or bed 26; when the wearer has last used a sanitizer dispenser 12 and/or a soap dispenser 12; the areas the user 16 and others have touched; gaps in personal protective equipment on themselves and others; and/or frequently touched areas. This information can be combined to create visual augmented reality feedback to indicate whether it is okay to proceed to touch or work near the patient.

This could be done through, for example:
- Color highlights over the patient. For example, an RTLS badge wearing patient or smart bed 26 is red when hands must be sanitized, and RTLS badge wearing patient or smart bed 26 has a green highlight when it is okay to approach and work near the patient from an infection prevention perspective.
- Visually, highlighting dispensers 12 on the way toward a patient when a disinfection must still occur
- Alerting staff to heightened infection prevention protocols for special patients, such as Methicillin-resistant Staphylococcus aureus (MRSA) cases. For example, soap dispensers 12 are highlighted instead of sanitizer dispensers 12 for certain infections. Reminders to wear certain PPE may also be provided.
- Alerting staff after touching a patient or patient surroundings, that they should not leave a room before sanitizing their hands. An example might be flashing the dispensers 12 in the area red, and/or flashing the exit in red.
- Alerting staff and/or patients to frequently touched areas, which appear as highlighted hotspots.
- Cleaning staff will automatically be alerted to clean areas that are frequently touched, and potential infection sources.
- Cleaning staff will also be alerted to dispensers 12 that are low on refills both through alerts that direct them to the dispensers 12, and visual information that appears when the dispenser 12 is seen.
- Smart germ sensing dispensers 12 could create augmented reality alerts that inform users 16 of new pathogens or dangerous pathogens on them or others in the area that require an escalation of hygiene protocols.
- Patients and visitors could be empowered with similar data, to ensure that they are also part of infection prevention efforts. Patients could also receive augmented reality data on staff that are treating them, highlighting them as red when they are not compliant with hygiene protocols.
- Augmented reality could be used to highlight the hands in red or shifting colors to green when they have been disinfected for the appropriate amount of time.
- Augmented reality could be used to call up visual guides to show how proper infection prevention techniques are to be used both for hand hygiene or cleaning tasks.
- Visually creating scores on infection prevention techniques, and creating a training task when scores fall below a certain level.

Additionally, users 16 could constantly or periodically see visual performance information on:
- time of last hygiene event;
- current individual compliance performance or missed opportunity counts;
- a significant drop in group compliance in the area; and/or
- a significant drop in available sanitizer in the area.

Additional available augmented reality information could include:
- Display fill level, type of product and expiration date of product in dispensers 12.
- For smart germ sensing dispensers 12, information on what germs have been detected recently on that dispenser 12, and other dispensers 12 in the area.

In one preferred embodiment, AR technology, combined with smart dispensers 12 and other smart sensors that create defined patient zones and track AR headset wearer movement, create an improved hand hygiene compliance feedback methodology within the AR headset field of vision, by alerting the wearer visually whether they are able to proceed to the patient because they have performed a required hygiene event.

The AR headset will create a visual cue overlayed on the patient zone to alert whether or not it is ok to proceed. Determining a compliant status will be achieved through information sent from RTLS technology on the headset wearer, tracking their movement through the facility, and smart dispensers 12, tracking and timestamping hygiene events, to understand when and where the headset wearer has performed a hygiene event. A software will then determine through this information, which zones are approachable, and relay this information to the headset. If the wearer has not met hand hygiene compliance criteria for a patient zone in their visual field, the AR headset will create a visual alert in the aforementioned patient zone (like a red highlight) and highlight nearby dispensers 12 in order to attract attention to them. The patient zone may, for example, be defined as a space extending half a meter from the patient or the patient's bed 26.

Additionally, the augmented reality system 10 could be used to improve janitorial / cleaning services in public and semi-public areas, optionally in combination with smart dispensers 12 and other sensors. For example:
- New tasks created from smart dispensers 12 and people counters may be automatically displayed in an augmented reality headset worn by cleaning staff.
- Optimal routes may be displayed visually in the AR headset to most efficiently direct closest staff to the correct area to complete a task flagged by smart dispensers 12 or sensors.
- Dispensers 12 that need refilling (as well as fill levels) are highlighted virtually in the AR headset.
- Identify cleaning surfaces that need to be cleaned, and correlate them to tasks that are generated in the software, automatically recording evidence of their completion.
- Visually highlight spots that have been missed while cleaning. For example, the system 10 may be configured to analyze video recorded by the augmented reality device 14 in order to identify areas that have been cleaned and that have not been cleaned, and the areas that have not been cleaned can be highlighted in the cleaner's visual field.
- Record which surfaces have been cleaned and provide feedback to the software.
- Illuminate what product is inside of each smart dispenser 12, what the fill level is, and/or what products are allowed/capable of being refilled into the dispenser 12.
- A "what to bring list" is visually displayed for each trip, as cleaning staff pack their carts or delivery vehicles.
- Automatically visually grading cleaning performance, and triggering an audit when scores are low.
- Be directed toward the nearest cleaning equipment.
- AR could identify when cleaning equipment is not used properly, or when cleaning patterns are insufficient. Proper cleaning motions could be displayed visually.
- New employees could be guided through a visual training program on how to use certain equipment properly in an AR space.
- AR could be used to show dispensers 12 are properly refilled, and what refills mate with the dispenser 12, or to identify incorrect refills.
- AR could be used to maintain and install dispensing equipment.

Although this disclosure has described and illustrated certain preferred embodiments of the invention, it is to be understood that the invention is not restricted to these particular embodiments. Rather, the invention includes all embodiments which are functional, electrical, or mechanical equivalents of the specific embodiments and features that have been described and illustrated herein.

## Claims

1. A system (10) comprising:
an augmented reality device (14) configured to present augmented reality content to a user (16); and
at least one sensor (20) configured to detect a hygiene event;
wherein the system (10) is configured to determine a hygiene status of the user (16) based at least in part on data collected by the at least one sensor (20); and
wherein at least some of the augmented reality content is selected based on the hygiene status of the user (16).

2. The system (10) according to claim 1, wherein the augmented reality content comprises visual content that is overlaid onto a visual field of the user (16).

3. The system (10) according to claim 2, wherein the visual content comprises a visual highlight (28) of a feature in the visual field of the user (16).

4. The system (10) according to claim 3, wherein the visual highlight (28) comprises a color that is overlaid onto the feature.

5. The system (10) according to claim 3 or claim 4, wherein the system (10) is configured to determine whether the user (16) is permitted to perform an action related to the feature based at least in part on the hygiene status of the user (16).

6. The system (10) according to claim 5, wherein, when the user (16) is permitted to perform the action, the visual highlight (28) is selected to inform the user (16) that the user (16) is permitted to perform the action.

7. The system (10) according to claim 5 or claim 6, wherein, when the user (16) is prohibited from performing the action, the visual highlight (28) is selected to inform the user (16) that the user (16) is prohibited from performing the action.

8. The system (10) according to any one of claims 5 to 7, wherein:
the feature comprises a physical space and the action comprises entering the physical space;
the feature comprises an object and the action comprises touching the object; or
the feature comprises a person and the action comprises touching the person.

9. The system (10) according to any one of claims 5 to 8, wherein the system (10) is configured to:
determine, based at least in part on the data collected by the at least one sensor (20), when the hygiene status of the user (16) has changed such that the user (16) is now permitted to perform the action related to the feature; and
modify the visual content to communicate to the user (16) that the user (16) is now permitted to perform the action.

10. The system (10) according to any one of claims 5 to 9, wherein, when the user (16) is prohibited from performing the action, the augmented reality device (14) is configured to produce an alert if the user approaches the feature; and
wherein the alert comprises an audible warning or a modification of the visual highlight (28).

11. The system (10) according to any one of claims 1 to 10, wherein the augmented reality device (14) comprises a headset that is worn by the user (16).

12. The system (10) according to any one of claims 1 to 11, wherein the at least one sensor (20) is incorporated into the augmented reality device (14).

13. The system (10) according to any one of claims 1 to 12, wherein the at least one sensor (20) is incorporated into a hand cleaning fluid dispenser (12).

14. The system (10) according to any one of claims 1 to 13, wherein the system (10) is configured to determine whether the user (16) should clean their hands; and
wherein, when the system (10) determines that the user (16) should clean their hands, the augmented reality content comprises a visual highlight (28) of a hand cleaning station in the visual field of the user (16).

15. The system (10) according to any one of claims 1 to 14, wherein the hygiene event comprises at least one of:
hand cleaning;
dispensing a hand cleaning fluid;
entering a patient room;
entering a bathroom;
touching a patient;
putting on personal protective equipment;
a failure to comply with a hand hygiene protocol; and
a failure to perform hand cleaning.
